# EUROPEAN PATENT SPECIFICATION

(11) **EP 0 356 236 B1**
(45) Date of publication and mention of the grant of the patent: **31.03.1993**
(21) Application number: 89308597.7
(22) Date of filing: 24.08.1989
(51) Int. Cl.: C07C 22/04, C07C 17/26, C07C 57/30

(54) **Chloroethylation of aromatic hydrocarbons**
Verfahren zur Chloroethylierung von aromatischen Kohlenwasserstoffen
Procédé pour la chloroéthylation d'hydrocarbures aromatiques

(30) Priority: 26.08.1988 US 240156
(43) Date of publication of application: 28.02.1990
(73) Proprietor: ETHYL CORPORATION, Baton Rouge Louisiana 70801 (US)
(72) Inventor: Knesel, George Albert, Orangeburg South Carolina 29115 (US)
(74) Representative: Collier, Jeremy Austin Grey

## Description

This invention relates to a process for chloroethylating aromatic hydrocarbons to form 1 chloro-1- arylethanes.

As disclosed in March, Advanced Organic Chemistry, Second Edition, McGraw-Hill, New York, 1977, pp. 501-502; Olah, Friedel-Crafts and Related Reactions, Volume 2, Interscience Publishers, New York, 1963-1964, pp. 659-784; U. S. Patent 2,516,971 (Ga- litzenstein et al.); and the references cited therein, it is known that aromatic compounds can be chloroalky- lated by reacting them with hydrogen chloride and an appropriate aldehyde in the presence of a Lewis acid or a proton acid as a catalyst, most commonly in the presence of zinc chloride.

The chloroalkylations utilizing formaldehyde as the aldehyde have been successfully employed in providing fairly high yields of 1-chloro-1-arylalkanes, and reasonably high yields of 1-chloro-1-arylalkanes have also been obtained from chloroalkylations utilizing higher aldehydes in some cases, e.g., when the aromatic compound has had an appropriate functional substituent or a plurality of alkyl substituents. However, when the aromatic compound has been a less reactive compound, e.g., an unsubstituted aromatic hydrocarbon or a monoalkylaromatic hydrocarbon, it has not been found possible to provide commercially acceptable yields of 1-chloro-1-arylalkane. There has been too much co-formation of diarylalkane by-product.

Another disadvantage of the known chloroalkylation processes utilizing the higher aldehydes has been their providing too much o-isomer in processes performed to provide 1-chloro-1- (4-alkylphenyl)alkanes, such as the compounds which have been synthesized by other techniques to provide intermediates for ibuprofen, related pharmaceuticals, or detergents. It would be desirable to find a way of increasing the para/ortho ratio obtainable from such processes to provide a more economical method of preparing the 1-chloro-1-(4-alkylphenyl)alkanes used in known processes, such as those of U. S. Patent 4,536,595 (Gardano et al.), Canadian Patent 1,197,254 (Francalanci et al.), British Patent 1,560,082 (Dynamit Nobel), Czechoslovakian Certificate of Authorship 219,752 (Palecek et al.), and Japanese Kokai 47-39050 (Miyatake et al.) and 52-111536 (Tokutake).

The present invention provides a process for chloroethylating aromatic hydrocarbons with hydrogen chloride and acetaldehyde to form 1-chloro-1- arylethanes. The new process reduces co-formation of diarylalkane by-product even when the aromatic hydrocarbon starting material is a monoalkylaromatic hydrocarbon. In addition when the aromatic hydrocarbon is a monoalkylbenzene, the new process gives a high para/ortho ratio in the product.

The process of the present invention comprises reacting an aromatic hydrocarbon with hydrogen chloride and acetaldehyde at a temperature in the range of-10°C to -35°C in the presence of at least 1.4 mols of hydrogen sulfate per mol of the aromatic hydrocarbon and less than 15% by weight of water, based on the weight of the hydrogen sulfate.

The aromatic hydrocarbon employed in the practice of the invention may be an unsubstituted aromatic hydrocarbon, such as benzene, naphthalene, anthracene, or phenanthracene. Also, it may be a poly- alkylaromatic hydrocarbon, such as xylene, pseudo- cumene, or mesitylene. However, because of the commercial interest in their chloroethylated products and the difficulty that has previously been encountered in preparing 1-chloro-1-arylethanes from them, the preferred aromatic hydrocarbons are monoalkylaromatic hydrocarbons, such as 1-methylnaphthalene, 2-methylnaphthalene, 9-methylanthracene, 9-butylanthracene, 9-dodecylanthracene, and the various monoalkylbenzenes, e.g., the methyl-, ethyl-, propyl-, isobutyl-, sec-butyl-, t-butyl-, isopentyl-, t-pentyl-, and hexylbenzenes. The most preferred aromatic hydrocarbons are the monoalkylbenzenes wherein the alkyl group contains 1-5 carbons.

The hydrogen chloride which is reacted with the aromatic hydrocarbon and acetaldehyde is preferably anhydrous or at least substantially anhydrous. However, some water in the hydrogen chloride can be tolerated as long as it is not an amount sufficient to raise the total amount of water in the reaction mixture above 15% by weight of the hydrogen sulfate, although it is preferred to keep the total amount of water at a concentration not higher than about 10% by weightofthe hydrogen sulfate. The hydrogen chloride may be incorporated into the reaction mixture per se or as a salt, such as sodium chloride, which reacts with sulfuric acid to form hydrogen chloride under the reaction conditions.

The acetaldehyde may be employed per se or may be introduced in the form of a substance, such as paraldehyde, which decomposes to yield acetaldehyde under the reaction conditions.

The aromatic hydrocarbon, hydrogen chloride, and acetaldehyde are normally employed in substantially equimolar amounts, but the proportions do not appear to be critical. Thus, amounts of any of the reactants which are smallerorlargerthan the equimolar amounts may be used if desired.

I order to avoid the presence of an excess of water in the reaction mixture, the hydrogen sulfate is introduced in the form of 85-98% sulfuric acid, preferably sulfuric acid having a concentration of 90-98%, most preferably 93-98%. The amount employed is such as to provide at least 1.4 mols, preferably at least 5 mols, per mol of aromatic hydrocarbon. There does not appear to be any maximum to the amount of hydrogen sulfate that may be used otherthan any maximum that might be imposed by economic constraints.

The reaction is conducted at a temperature in the range of-10°C to -35°C, preferably -25°C to -35°C, in order to achieve the advantages of the invention.

The process of the invention is exothermic, so the reactants should be combined at a rate that permits control of the reaction temperature. In conducting the process it is preferred to add a mixture of the aromatic hydrocarbon and acetaldehyde to a sulfuric acid solution saturated with hydrogen chloride and to add additional hydrogen chloride during the reaction. However, alternatively, the acetaldehyde and hydrogen chloride can be prereacted, or the aromatic hydrocarbon can be the first charge to the reaction vessel.

The invention is useful as an alternative method of preparing 1-chloro-1-arylethanes from aromatic hydrocarbons that are known to be capable of providing high yields of such products by known chloroethylation techniques. However, it is particularly advantageous as a method of preparing 1-chloro-1- arylethanes from the less reactive aromatic hydrocarbons, such as monoalkylbenzenes, that have not previously been found to be capable of providing high yields of such products by chloroalkylation processes utilizing acetaldehyde. The process is of especial interest in the chloroethylation of monoalkylbenzenes, where it has the advantage of not only minimizing the co-formation of diarylalkane by-product but of also increasing the para/ortho ratio in the product.

As is known, the products obtained by the process are useful as internal standards or as intermediates for the preparation of monomers, detergents, or pharmaceuticals. When they are used as chemical intermediates, they may be subjected to the same reactions as have previously been used to convert them to desired products. For example, the 1-chloro-1-arylethanes can be dehydrochlorinated in any known manner to provide styrenes which can then be polymerized by known techniques.

A particularly interesting application of the 1 chloro- 1-(4-alkylphenyl)ethanes which are prepared in a preferred embodiment of the invention is as intermediates for the preparation of ibuprofen and related pharmaceuticals. When they are used in such applications, they may be converted to the desired products in any suitable manner. For example, they may be reacted with carbon monoxide in the presence of a carbonylation catalyst and then acidified to the corresponding propionic acids as in Gardano et al., Francalanci et al., or Dynamit Nobel, or they may be reacted with an alkali metal cyanide or a tetraalkylammonium cyanide and then hydrolyzed to the corresponding propionic acids as in Palecek et al. or Tokutake. Another useful synthesis involves reacting the compounds with magnesium, carbonating the resultant Grignard reagent with carbon dioxide, and hydrolyzing the carbonated product to the propionic acid as in Miyatake et al.

The following examples are given to illustrate the invention and are not intended as a limitation thereof.

### EXAMPLE I

A suitable reaction vessel was charged with 60 mL of 93% sulfuric acid, which was cooled to -35°C and saturated with anhydrous hydrogen chloride. A solution of 7.8g of acetaldehyde and 21.3g of isobutylbenzene was fed to the reaction vessel over a period of 30 minutes at -35°C with hydrogen chloride bubbling into the reaction mass. The reaction mass was stirred for one hourat-35°Cand then poured into ice water. Analysis showed an 88/12 molar ratio of 1-chloro-l-(isobutylphenyl)ethane to 1,1-di-(isobutylphenyl)ethane.

### EXAMPLE 11

Crude 1-chloro-1-(isobutylphenyl)ethane containing 47g of 1-chloro-1-(isobutylphenyl)ethane was added to a mixture of 17g of sodium cyanide in 126g of dimethyl sulfoxide (DMSO). The reaction mixture was heated to 80°C with agitation for 10 hours, after which the DMSO and inorganic salts were removed by water washing to yield 1-cyano-1-(isobutyiphenyi) ethane. The crude nitrile was reacted with excess 50% sodium hydroxide at 135°C for four hours to form the sodium salt of 2-(isobutylphenyl)-propionic acid, which was then acidified and crystallized from hexane at -10°C. The para/ortho ratio of the resultant 2-(isobutylphenyl)propionic acid in hexane solution was approximately 200/1.

## Claims

1. A process for producing a 1-chloro-1-arylethane which comprises reacting an aromatic hydrocarbon with hydrogen chloride and acetaldehyde at a temperature in the range of -10°C to -35°C in the presence of at least 1.4 mols of hydrogen sulfate per mol of the aromatic hydrocarbon and less than 15% by weight of water, based on the weight of the hydrogen sulfate.

2. The process of claim 1 wherein the aromatic hydrocarbon is a monoalkylbenzene in which the alkyl contains 1 to 5 carbon atoms and the product is a 1-chloro-1-(4-alkylphenyl)ethane.

3. The process of claim 1 wherein isobutylbenzene is reacted with anhydrous hydrogen chloride and acetaldehyde at a temperature in the range of - 25°C to -35°C in the presence of at least 5 mols of hydrogen sulfate per mol of isobutyl benzene, the hydrogen sulfate being introduced in the form of 93-98% sulfuric acid, and 1-chloro-1-(4-isobutylphenyl)ethane is obtained.

4. A process for preparing a 2-(4-alkylphenyl)propionic acid which comprises reacting a 1-chloro-1-(4-alkylphenyl)-ethane produced by the process of claim 2 with carbon monoxide in the presence of a carbonylation catalyst and acidifying the product.

5. The process of claim 4 wherein the starting material is 1-chloro-1-(4-isobutylphenyl)ethane produced by the process of claim 3.

6. A process for preparing a 2-(4-alkylphenyl)propionic acid which comprises reacting a 1-chloro-1-(4-alkylphenyl)-ethane produced by the process of claim 2 with an alkali metal cyanide or tetraalkylammonium cyanide and hydrolysing the product.

7. The process of claim 6 wherein the starting material is 1-chloro-1-(4-isobutylphenyl)ethane produced by the process of claim 3.

## Patentansprüche

1. Verfahren zur Herstellung eines 1-Chlor-1- arylethans, welches das Umsetzen eines aromatischen Kohlenwasserstoffs mit Chlorwasserstoff und Acetaldehyd bei einer Temperatur im Bereich von -10°C bis -35°C in Gegenwart von wenigstens 1,4 Mol Hydrogensulfat pro Mol des aromatischen Kohlenwasserstoffs und weniger als 15 Gew.-% Wasser, bezogen auf das Gewicht des Hydrogensulfats, umfaßt.

2. Verfahren nach Anspruch 1, worin der aromatische Kohlenwasserstoff ein Monoalkylbenzol ist, in dem die Alkylgruppe 1 bis 5 Kohlenstoffatome enthält, und das Produkt ein 1-Chlor-1-(4-alkylphenyl-)ethan ist.

3. Verfahren nach Anspruch 1, worin Isobutylbenzol mit wasserfreiem Chlorwasserstoff und Acetaldehyd bei einer Temperatur im Bereich von -25°C bis-35°C in Gegenwart von wenigstens 5 Mol Hydrogensulfat pro Mol Isobutylbenzol umgesetzt wird, wobei das Hydrogensulfat in Form von 93- bis 98%-iger Schwefelsäure zugeführt wird, und 1-Chlor-1-(4-isobutylphenyl-)ethan erhalten wird.

4. Verfahren zur Herstellung einer 2-(4-Alkylphenyl-) propionsäure, welches die Umsetzung eines 1-Chlor-1-(4-alkylphenyl-)ethans, das durch das Verfahren nach Anspruch 2 hergestellt wurde, mit Kohlenmonoxid in Gegenwart eines Carbonylierungskatalysators und Acidifizieren des Produkts umfaßt.

5. Verfahren nach Anspruch 4, worin das Ausgangsmaterial 1-Chlor-1-(4-isobutylphenyl)-ethan ist, das durch das Verfahren nach Anspruch 3 hergestellt wurde.

6. Verfahren zur Herstellung einer 2-(4-Alkylphenyl-) propionsäure, welches die Umsetzung eines 1-Chlor-1-(4-alkylphenyl-)ethans, das durch das Verfahren nach Anspruch 2 hergestellt wurde, mit einem Alkalimetallcyanid oder Tetraalkylammoniumcyanid und die Hydrolyse des Produkts umfaßt.

7. Verfahren nach Anspruch 6, worin das Ausgangsmaterial 1-Chlor-1-(4-isobutylphenyl)-ethan ist, das durch das Verfahren nach Anspruch 3 hergestellt wurde.

## Revendications

1. Procédé de production d'un 1-chloro-1-arylétha- ne, qui comprend la réaction d'un hydrocarbure aromatique avec le chlorure d'hydrogène et l'acétaldéhyde à une température comprise dans la plage de -10°C à -35°C en présence d'au moins 1,4 mole de sulfate d'hydrogène par mole d'hydrocarbure aromatique et de moins de 15 % en poids d'eau, sur la base du poids de sulfate d'hydrogène.

2. Procédé suivant la revendication 1, dans lequel l'hydrocarbure aromatique est un monoalkylben- zène dont le radical alkyle contient 1 à 5 atomes de carbone, et le produit est un 1-chloro-1-(4-alkylphényl)éthane.

3. Procédé suivant la revendication 1, dans lequel on fait réagir de l'isobutylbenzène avec du chlorure d'hydrogène anhydre et de l'acétaldéhyde à une température comprise dans la plage de - 25°C à -35°C en présence d'au moins 5 moles de sulfate d'hydrogène par mole d'isobutylbenzène, le sulfate d'hydrogène étant introduit sous forme d'acide sulfurique à 93-98 %, et on obtient du 1-chloro-1-(4-isobutylphényl)éthane.

4. Procédé de production d'un acide 2-(4-alkylphé- nyl)propionique, qui comprend la réaction d'un 1-chloro-1-(4-alkylphényl)-éthane produit par le procédé de la revendication 2 avec l'oxyde de carbone en présence d'un catalyseur de carbonylation, et l'acidification du produit.

5. Procédé suivant la revendication 4, dans lequel la matière de départ est le 1-chloro-1-(4-isobutyl- phényl)éthane produit par le procédé suivant la revendication 3.

6. Procédé de production d'un acide 2-(4-alkylphé- nyl)propionique, qui comprend la réaction d'un 1-chloro-1-(4-alkylphényl)-éthane produit par le procédé suivant la revendication 2, avec un cyanure de métal alcalin ou un cyanure de tétra-alky- lamonium, et l'hydrolyse du produit.

7. Procédé suivant la revendication 6, dans lequel la matière de départ est le 1-chloro-1-(4-isobutyl- phényl)éthane produit par le procédé de la revendication 3.
